# EUROPEAN PATENT APPLICATION

(11) **EP 3 919 015 A1**
(43) Date of publication of application: **08.12.2021**
(21) Application number: 21165828.1
(22) Date of filing: 30.03.2021
(51) Int. Cl.: A61B 18/14

(54) **SMOOTH-EDGE AND EQUIDISTANTLY SPACED ELECTRODES ON AN EXPANDABLE FRAME OF A CATHETER FOR IRREVERSIBLE-ELECTROPORATION (IRE)**

(30) Priority: 04.06.2020 US 202016892514
(71) Applicant: Biosense Webster (Israel) Ltd, Yokneam, 2066717 (IL)
(72) Inventor: GOVARI, Assaf, 2066717 Yokneam (IL); BEECKLER, Christopher Thomas, Irwindale, CA 91706 (US); KEYES, Joseph Thomas, Irwindale, CA 91706 (US); HERRERA, Kevin Justin, Irwindale, CA 91706 (US)
(74) Representative: Carpmaels & Ransford LLP

(57) **Abstract**

A system includes a catheter and an IRE pulse generator. The catheter includes an expandable frame fitted at a distal end thereof, the expandable frame including multiple electrodes configured to be placed in contact with a tissue in an organ of a patient, wherein a lateral distance between neighboring edges of any pair of adjacent electrodes is uniform along a longitudinal axis, and wherein the electrodes are configured to apply irreversible electroporation (IRE) pulses to tissue between pairs of the electrodes. The IRE pulse generator is configured to generate the IRE pulses.

## Description

### FIELD OF THE INVENTION

The present invention relates generally to invasive medical probes, and particularly to catheters comprising expandable frames for irreversible electroporation (IRE).

### BACKGROUND OF THE INVENTION

Various multi-electrode catheter geometries were previously proposed in the patent literature. For example, U.S. Patent Application Publication No. 2018/0280080 describes an inflatable balloon fitted at a distal end of a probe. When the balloon is deployed and inflated inside a body cavity, a distal pole on a distal side of the balloon is brought into contact with cavity wall tissue. While ablation electrodes of the balloon are in contact with tissue, there are gaps (i.e., open spaces) on the balloon that are not covered by the ablation electrodes. In operation, heat in the tissue that is generated in response to ablation energy delivered by the ablation electrodes (i.e., that surround the open spaces) is conducted to nearby tissue and ablates the tissue that is in contact with any gaps that are in contact with tissue.

As another example, U.S. Patent Application Publication No. 2013/0304054 describes methods and catheter apparatus for non-continuous circumferential treatment of a body lumen using inflatable balloons. The apparatus may be positioned within a body lumen of a patient and may deliver energy at a first lengthwise and angular position to create a less-than-full circumferential treatment zone at the first position. The apparatus may also deliver energy at one or more additional lengthwise and angular positions within the body lumen to create less-than-full circumferential treatment zone(s) at the one or more additional positions that are offset lengthwise and angularly from the first treatment zone. Superimposition of the first treatment zone and the one or more additional treatment zones defines a non-continuous circumferential treatment zone without formation of a continuous circumferential lesion.

U.S. Patent Application Publication No. 2019/0183567 describes a medical instrument that includes a shaft, an inflatable balloon and radiofrequency (RF) ablation electrodes. The shaft is configured for insertion into a body of a patient. The inflatable balloon is coupled to a distal end of the shaft. The radiofrequency (RF) ablation electrodes are disposed on an external surface of the balloon, each electrode having a distal edge configured to reduce electric field angular gradients of an RF electric field emitted from the distal edge.

### SUMMARY OF THE INVENTION

An embodiment of the present invention that is described hereinafter provides a system includes a catheter and an IRE pulse generator. The catheter includes an expandable frame fitted at a distal end thereof, the expandable frame including multiple electrodes configured to be placed in contact with tissue in an organ of a patient, wherein a lateral distance between neighboring edges of any pair of adjacent electrodes is uniform along a longitudinal axis, and wherein the electrodes are configured to apply irreversible electroporation (IRE) pulses to tissue between pairs of the electrodes. The IRE pulse generator is configured to generate the IRE pulses.

In some embodiments, the expandable frame includes a membrane of an expandable balloon.

In some embodiments, the electrodes are disposed equiangularly about the longitudinal axis of the distal end.

In an embodiment, the catheter is configured to apply the IRE pulses between adjacent electrodes.

In some embodiments, the organ is a heart and the tissue is a pulmonary vein (PV) ostium tissue.

There is additionally provided, in accordance with another embodiment, a method including placing multiple electrodes of a catheter in contact with tissue in an organ of a patient, the catheter including an expandable frame fitted at a distal end thereof, the expandable frame including the multiple electrodes, wherein a lateral distance between neighboring edges of any pair of electrodes is uniform along a longitudinal axis. Irreversible electroporation (IRE) pulses are applied to between one or more pairs of the electrodes.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a catheter-based irreversible electroporation (IRE) system, in accordance with an exemplary embodiment of the present invention; and
Fig. 2 is a flow chart that schematically illustrates a method for applying irreversible electroporation (IRE) pulses using the IRE balloon catheter of Fig. 1, in accordance with an exemplary embodiment of the present invention.

### DETAILED DESCRIPTION OF EMBODIMENTS

### OVERVIEW

Irreversible electroporation (IRE), also called Pulsed Field Ablation (PFA), may be used as an invasive therapeutic modality to kill tissue cells by subjecting them to high-voltage pulses. Specifically, IRE pulses have a potential use to kill myocardium tissue cells in order to treat cardiac arrhythmia. Of particular interest is the use of bipolar electric pulses (e.g., using a pair of electrodes in contact with tissue) to kill tissue cells between the electrodes. Cellular destruction occurs when the transmembrane potential exceeds a threshold, leading to cell death and thus the development of a tissue lesion.

Cardiac IRE ablation may be performed using an expandable frame (e.g., balloon or basket) fitted on a distal end of an ablation catheter. The expandable frame, which is fitted with ablation electrodes, is navigated through the cardiovascular system and inserted into a heart in order to, for example, ablate an ostium of a pulmonary vein (PV).

However, not every electrode shape may be suited for applying high voltage IRE bipolar pulses. For example, preferential current density at sharp edges of the ablation electrodes should be avoided in IRE ablation in order to avoid excessive heat generation or electrical arcing in those regions. Moreover, as the applied electrical field should be above a certain threshold level to be effective, variable inter-electrode distances between adjacent electrodes, which results in variable strength of the electric field, may lead to an unpredictable clinical outcome of an IRE balloon ablation procedure.

Exemplary embodiments of the present invention that are described hereinafter provide IRE ablation methods and systems that use an expandable frame (e.g., a balloon membrane) having electrodes optimized for IRE ablation. A disclosed balloon comprises smooth edged electrodes disposed equiangularly over the expandable frame (e.g., the membrane of the balloon). The expandable frame shape is designed with a flattened distal portion to enable approximately constant distance between electrodes, even for the electrode parts covering the distal portion of the frame. This approximately uniform inter-electrode distance (i.e., a lateral distance between neighboring edges of any pair of adjacent electrodes being approximately uniform along the longitudinal axis) allows for the application of consistent electric field strengths between adjacent electrodes while minimizing any undesired thermal effects. Additionally, electrodes may be selected so that the electric field may be generated between any combination of electrodes and not just adjacent electrodes.

There are numerous ways to achieve approximately uniform inter-electrode distance, which should be considered covered by this application. In one example, a balloon shown below has a flattened shape of the distal portion of the balloon, to maintain a distance between adjacent electrodes approximately constant even where electrodes cover the distal portion of the balloon. As another example, electrodes can be formed in a two-dimensional array having approximately equal distances between adjacent electrodes of the array along mutually orthogonal directions between the electrodes, e.g., using rhombus shaped electrodes that are narrower where the expendable frame has a diminishing radius, towards distal and proximal ends of the expendable frame.

In one exemplary embodiment, an expandable balloon in use has a small diameter, e.g., between 9-12 mm, that reduces balloon creasing when stowed. The disclosed flattened shape of the balloon membrane enables the balloon to support the aforementioned electrodes which are still sufficiently long and large to be used to apply the required approximately uniform electric field magnitude between adjacent electrodes.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

In another exemplary embodiment, a processor of the system is used by a physician to select (e.g., to upload from a memory) an ablation protocol that specifies parameters of the IRE pulses to be applied by the multiple pairs of adjacent electrodes. Using the disclosed balloon, these parameters are optimized, for example, to kill myocardium cells of a PV ostium with minimal or no collateral damage, and thus improve the clinical outcome of an IRE treatment of cardiac arrhythmia.

Using one of the disclosed expandable frames (e.g., an expandable balloon) to apply cardiac IRE ablation having approximately uniform IRE field strengths may lead to more predictable, and thus safer and more effective, multi-electrode cardiac IRE ablation.

### SMOOTH EDGED EQUIDISTANTLY SPACED ELECTRODES IN BALLOON CATHETER FOR IRE

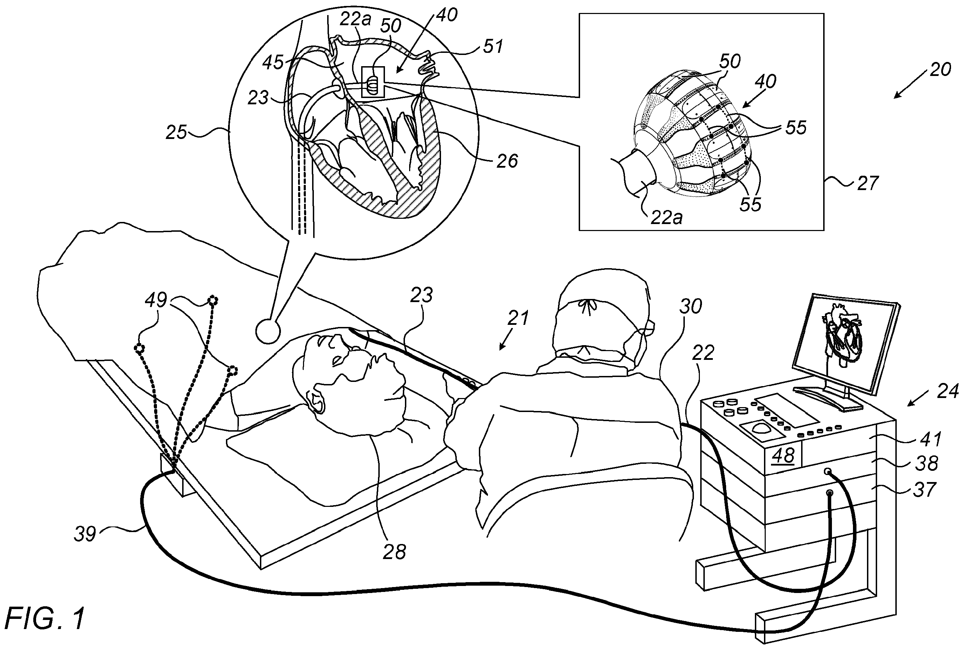

Fig. 1 is a schematic, pictorial illustration of a catheter-based irreversible electroporation (IRE) system 20, in accordance with an exemplary embodiment of the present invention. System 20 comprises a catheter 21, wherein a shaft 22 of the catheter is inserted by a physician 30 through the vascular system of a patient 28 through a sheath 23. The physician 30 then navigates a distal end 22a of shaft 22 to a target location inside a heart 26 of the patient 28.

Once distal end 22a of shaft 22 has reached the target location, physician 30 retracts sheath 23 and expands balloon 40, typically by pumping saline into balloon 40. Physician 30 then manipulates shaft 22 such that electrodes 50 disposed on balloon 40 engage an interior wall of a PV ostium 51 to apply high-voltage IRE pulses via electrodes 50 to ostium 51 tissue. Physician 30 may use balloon 40 to engage any part, interior or exterior, of heart 26 of the patient 28.

As seen in insets 25 and 27, distal end 22a is fitted with an expandable balloon 40 comprising multiple equidistant smooth-edged IRE electrodes 50. Due to the flattened shape of the distal portion of balloon 40, a distance 55 between adjacent electrodes 50 remains approximately constant even where electrodes 50 cover the distal portion. Balloon 40 configuration therefore allows more effective (e.g., with approximately uniform electric field strength) electroporation between adjacent electrodes 50 while the smooth edges of electrodes 50 minimize unwanted thermal effects.

Certain aspects of inflatable balloons are addressed, for example, in U.S. Provisional Patent Application No. 62/899,259, filed September 12, 2019, titled "Balloon Catheter with Force Sensor," and in U.S. Patent Application No. 16/726,605, filed December 24, 2019, titled, "Contact Force Spring with Mechanical Stops," which are both assigned to the assignee of the present patent application and whose disclosures are incorporated herein by reference.

In the exemplary embodiment described herein, catheter 21 may be used for any suitable diagnostic purpose and/or therapeutic purpose, such as electrophysiological sensing and/or the aforementioned IRE isolation of PV ostium 51 tissue in left atrium 45 of heart 26.

The proximal end of catheter 21 is connected to a console 24 comprising an IRE pulse generator 38 configured to apply the IRE pulses between adjacent electrodes 50. The electrodes are connected to IRE pulse generator 38 by electrical wiring running in shaft 22 of catheter 21. A memory 48 of console 24 stores IRE protocols comprising IRE pulse parameters, such as peak bipolar voltage and pulse width.

Console 24 comprises a processor 41, typically a general-purpose computer, with suitable front end and interface circuits 37 for receiving signals from catheter 21 and from external electrodes 49, which are typically placed around the chest of patient 28. For this purpose, processor 41 is connected to external electrodes 49 by wires running through a cable 39.

During a procedure, system 20 can track the respective locations of electrodes 50 inside heart 26, using the Advanced Catheter Location (ACL) method, provided by Biosense-Webster (Irvine California), which is described in US Patent No. 8,456,182, whose disclosure is incorporated herein by reference.

Processor 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

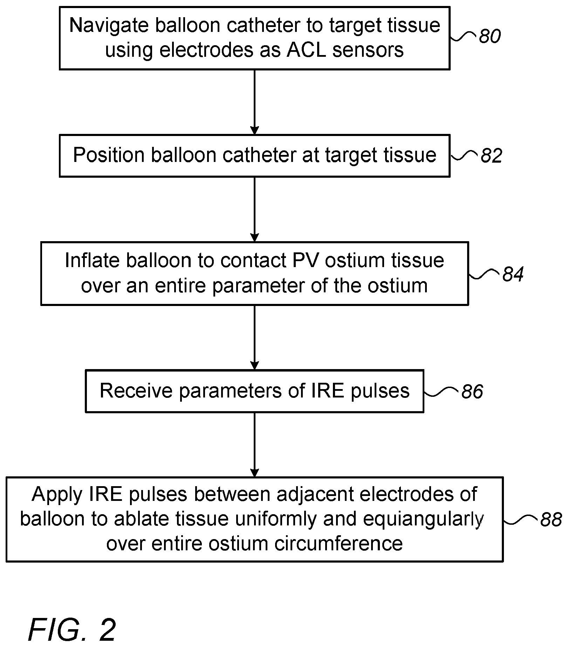

In particular, processor 41 runs a dedicated algorithm as disclosed herein, including the algorithm flow-charted in Fig. 2, that enables processor 41 to perform the disclosed steps, as further described below. In particular, processor 41 is configured to command IRE pulse generator 38 to output IRE pulses according to a treatment protocol that processor 41 uploads from memory 48.

An example of IRE ablation settings in a protocol that may be used for ablating cardiac tissue using the disclosed balloon 40 are given in Table I, below.

**Table I**

| **Parameter** | **Range** |
|---|---|
| Preset IRE peak voltage | 500-2000 V |
| Pulse width | 0.5-10 micro second |
| Repetition rate | 1-400 Hz |
| Number of pulses | 10-200 |

Fig. 2 is a flow chart that schematically illustrates a method for applying IRE pulses using balloon 40 of Fig. 1, in accordance with an exemplary embodiment of the present invention. The algorithm, according to the exemplary embodiment, carries out a process that begins when physician 30 navigates the balloon catheter to a target tissue location in an organ of a patient, such as at PV ostium 51, using, for example, electrodes 50 as ACL sensing electrodes, at a balloon catheter navigation step 80.

Next, physician 30 positions the balloon catheter at ostium 51, at a balloon catheter positioning step 82. Next, physician 30 fully inflates balloon 40 to contact target tissue with electrodes 50 over an entire circumference of PV ostium 51, at a balloon inflation step 84.

Next, at an IRE planning step 86, processor 41 uploads a protocol with parameters of the IRE pulses to apply to tissue, such as given in Table I.

Using the IRE pulse parameters, processor 41 commands generator 38 to apply the IRE pulses to tissue, at an IRE treatment step 88. The smooth edged, uniformly distant and equiangular electrodes 50 of balloon 40 enable the application of the IRE pulses between adjacent electrodes to isolate an arrhythmia fully, i.e., over an entire circumference of ostium 51.

Although the exemplary embodiments described herein mainly address cardiac applications, the methods and systems described herein can also be used in other medical applications, such as in neurology, otolaryngology, and general surgery.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A system for irreversible electroporation, the system comprising:
a catheter comprising an expandable frame fitted at a distal end thereof, the expandable frame comprising multiple electrodes configured to be placed in contact with tissue in an organ of a patient, wherein a lateral distance between neighboring edges of any pair of adjacent electrodes is uniform along a longitudinal axis, and wherein the electrodes are configured to apply irreversible electroporation (IRE) pulses to tissue between pairs of the electrodes; and
an IRE pulse generator, which is configured to generate the IRE pulses.

2. The system according to claim 1, wherein the expandable frame comprises a membrane of an expandable balloon.

3. The system according to claim 1, wherein the electrodes are disposed equiangularly about the longitudinal axis of the distal end.

4. The system according to claim 1, wherein the catheter is configured to apply the IRE pulses between adjacent electrodes.

5. The system according to claim 1, wherein the organ is a heart and the tissue is a pulmonary vein (PV) ostium tissue.

6. A method for irreversible electroporation, the method comprising:
placing multiple electrodes of a catheter in contact with tissue in an organ of a patient, the catheter comprising an expandable frame fitted at a distal end thereof, the expandable frame comprising the multiple electrodes, wherein a lateral distance between neighboring edges of any pair of electrodes is uniform along a longitudinal axis; and
applying irreversible electroporation (IRE) pulses between one or more pairs of the electrodes.

7. The method according to claim 6, wherein the expandable frame comprises a membrane of an expandable balloon.

8. The method according to claim 6, wherein the electrodes are disposed equiangularly about the longitudinal axis of the distal end.

9. The method according to claim 6, wherein the organ is a heart and the tissue is a pulmonary vein (PV) ostium tissue.
